# EUROPEAN PATENT SPECIFICATION

(11) **EP 2 198 790 B1**
(45) Date of publication and mention of the grant of the patent: **16.03.2016**
(21) Application number: 10156522.4
(22) Date of filing: 25.07.2008
(51) Int. Cl.: A61B 17/28, A61B 17/00

(54) **Operating mechanism, medical manipulator, and surgical robot system**
Betriebsmechanismus, medizinisches Manipuliergerät und chirurgisches Robotersystem
Mécanisme de fonctionnement, manipulateur médical, et système de robot chirurgical

(30) Priority: 25.07.2007 JP 2007193527; 16.04.2008 JP 2008106818
(43) Date of publication of application: 23.06.2010
(62) Divisional of application: 08161162.6
(73) Proprietor: Karl Storz GmbH & Co. KG, 78532 Tuttlingen (DE)
(72) Inventor: Omori, Shigeru, Ashigarakami-gun Kanagawa 259-0151 (JP); Uenohara, Shuichi, Fujinomiya-shi Shizuoka 418-0015 (JP); Sano, Hiroaki, Fujinomiya-shi Shizuoka 418-0015 (JP)
(74) Representative: TBK

(56) References cited:
- EP-A2- 1 110 557
- WO-A1-96/06566
- WO-A1-97/15334
- US-A- 5 361 583
- US-A- 5 730 742
- US-A- 5 770 739
- US-A- 6 007 515
- US-A1- 2002 068 012
- US-A1- 2006 217 697
- US-B1- 6 245 091

## Description

### BACKGROUND OF THE INVENTION

### Field of the Invention:

The present invention relates to a method for sterilizing a grip handle of a medical manipulator according to the preamble of the claim, the features of which are known from document US 5 730 742. Document US5361583 discloses a pressurized fluid actuation system with variable force and stroke output for use in a surgical instrument.

### Description of the Related Art:

In laparoscopic surgery, a number of small holes are opened in a patient's abdomen or the like, and an endoscope, a forceps (or manipulator) or the like is inserted, and surgery is carried out while the surgeon observes an image from the endoscope on a monitor. In this type of laparoscopic surgery, owing to the fact that opening of the abdominal cavity is unnecessary, the burden on the patient is small, and the number of days required for the postoperative recovery and the number of days spent in the hospital can be significantly reduced. Therefore, laparoscopic surgical operations are expected to find an increased range of applications.

As disclosed in Japanese Laid-Open Patent Publication No. 2004-105451 and Japanese Laid-Open Patent Publication No. 2002-102248, for example, a manipulator system comprises a manipulator and a controller for controlling the manipulator. The manipulator comprises an operating unit which is manually operable and a working unit replaceably mounted on the operating unit.

The working unit (instrument) comprises a long joint shaft and a distal-end working unit (also referred to as an end effector) mounted on the distal end of the joint shaft. The operating unit has actuators (motors) for actuating the working unit through wires. The wires have proximal end portions wound around respective pulleys. The controller energizes the motors of the operating unit to cause the pulleys to move the wires back and forth.

The working unit is constructed so as to be detachable with respect to the operating unit in order to enable cleaning to be carried out easily following completion of a surgical technique. Further, in laparoscopic surgery, various different types of working units are used depending on the surgery involved. A gripper, scissors, an electrical knife, an ultrasonic knife, a surgical drill or the like may be given as examples thereof. From the standpoint of being able to exchange these working units, a structure in which the working unit is detachable with respect to the operating unit also is beneficial.

There has been proposed a medical robot system including medical manipulators movable by robot arms (see U.S. patent No. 6331181, for example). The medical robot system can be remotely controlled by a master arm and can be operated in various ways under programmed control.

The medical robot system has a plurality of robot arms that are selectively used to perform respective surgical techniques. One of the robot arms supports an endoscope for capturing an image in a body cavity which is to be confirmed on a display monitor.

If the manually operable operating unit of the manipulator has a grip handle, then the operating unit can reliably be gripped and operable by hand. If the grip handle includes a trigger lever movable back and forth, then a gripper or scissors mounted on the working unit can accurately be opened and closed by an index finger on the trigger lever.

General trigger levers are used to determine a certain timing to start a desired action just like the trigger level of a pistol determines a timing to fire a bullet. However, they may not necessarily be suitable to open and close a gripper or scissors with delicate movements.

The operating unit of the manipulator also includes a switch for activating or suspending the system in addition to a trigger lever for operating the distal-end working unit. Therefore, the switch and the trigger lever need to be positioned for being easily manipulatable by the operator.

The grip handle is of a hollow structure housing therein electric components such as a sensor for detecting the displacement of the trigger lever. There has been a demand for grip handles which securely protect electric components housed therein and whose hollow structures can simply be produced.

Furthermore, as a manipulator employs a variety of working units, the working units should desirably be detachably mounted on the operating unit, as described above. While the manipulator is in operation, it is desirable that the working unit which is mounted on the operating unit should securely be held in position by the operating unit.

An operating mechanism of the actuators and the controller are connected to each other by a cable. Forces that are produced by the cable should not adversely affect the manner in which the manipulator operates. The operating mechanism should preferably be compact for better operability.

The distal-end working units of some medical manipulators include a rolling mechanism rotatable coaxially with the joint shaft for enabling the working unit to make complex motions. However, the rolling mechanism may have its angular displacement difficult to recognize.

The distal-end working units of some other medical manipulators include a plurality of angularly moving mechanisms angularly movable about axes not parallel to the joint shaft for enabling the distal-end working unit to make more complex motions. However, if the distal-end working unit operated by one or more of the angularly moving mechanisms remains in an attitude not parallel to the joint shaft, then care should be taken to correct the attitude of the distal-end working unit when pulling out the working unit because the distal end-working unit would otherwise interfere with the trocar on the patient.

### SUMMARY OF THE INVENTION

It is the object of the invention, to provide an improved method for sterilizing a grip handle.

The object of the invention is achieved by a method for sterilizing a grip handle according to claim 1.

The above and other objects, features, and advantages of the present invention will become more apparent from the following description when taken in conjunction with the accompanying drawings in which preferred embodiments of the present invention are shown by way of illustrative example.

### BRIEF DESCRIPTION OF THE DRAWINGS

FIG. 1 is a perspective view of a manipulator having a grip handle to be sterilized according to an embodiment of the present invention;
FIG. 2 is a side elevational view of the manipulator with a working unit and an operation command unit being separate from each other;
FIG. 3 is a perspective view of a distal-end working unit;
FIG. 4 is a view showing the surface of a composite input unit;
FIG. 5 is a perspective view of the operation command unit as an operating mechanism.
FIG. 6 is a sectional plan view of a trigger lever;
FIG. 7 is a side elevational view of another operation command unit;
FIG. 8 is an enlarged fragmentary side elevational view of a trigger lever, a switch, and nearby parts of the operation command unit shown in FIG. 7;
FIG. 9 is a sectional side elevational view of a check valve;
FIG. 10 is a perspective view, as seen obliquely from below, of a connector of the working unit and an actuator block of the operation command unit which are separate from each other;
FIG. 11 is a front elevational view of the manipulator, showing the manner in which two pusher surfaces are simultaneously pressed to detach the connector from the actuator block;
FIG. 12 is a side elevational view of a modified manipulator;
FIG. 13 is a perspective view of another modified manipulator;
FIG. 14 is a view showing the surface of a composite input unit with a third switch disposed in a shuttle ring;
FIG. 15 is a view showing the surface of a composite input unit with the third switch disposed outside the shuttle ring;
FIG. 16 is a perspective view of still another manipulator; and
FIG. 17 is a schematic perspective view of a surgical robot system with a working unit connected to the distal end of a robot arm.

### DESCRIPTION OF THE PREFERRED EMBODIMENTS

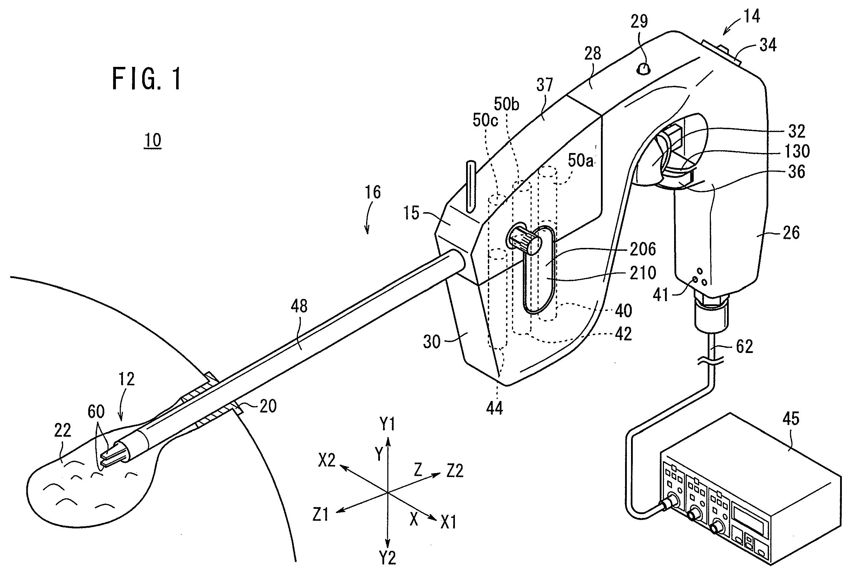

A method for sterilizing a grip handle of a medical manipulator according to the present invention will be described below an the basis of FIGS. 1 through 17 of the accompanying drawings.

A manipulator 10 (see FIG. 1) has a distal-end working unit 12 for gripping a portion of a living tissue, a curved needle, or the like for performing a certain surgical treatment, and is usually referred to as gripping forceps or a needle driver (needle holder).

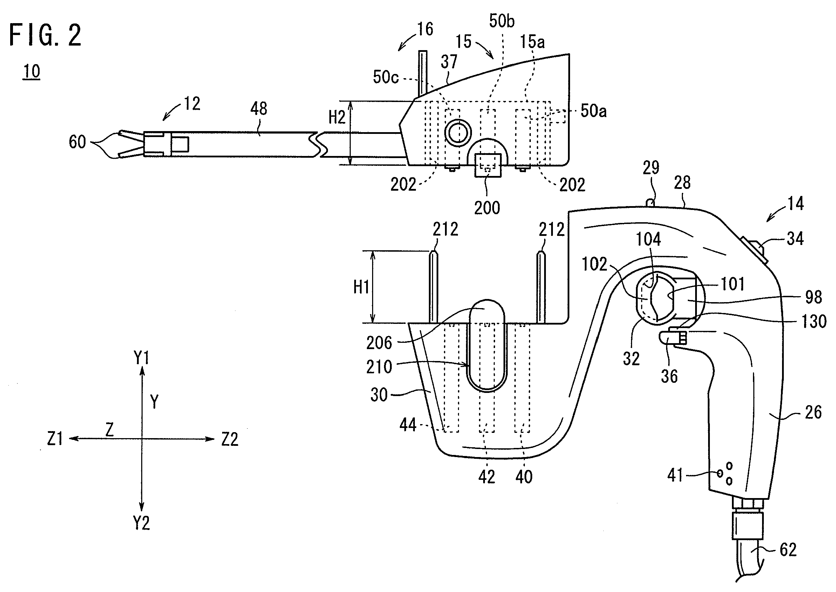

As shown in FIGS. 1 and 2, the manipulator 10 comprises an operation command unit (operating mechanism) 14 on a proximal end portion which is held and operated by hand and a working unit 16 detachably mounted on the operation command unit 14.

It shall be assumed in the following description that, as shown in FIG. 1, the transverse directions are defined as X directions, the vertical directions as Y directions, and the longitudinal directions of a hollow joint shaft 48 as Z directions. Further, among the X directions, the rightward direction is defined as an X1 direction, and the leftward direction as an X2 direction, among the Y directions, the upward direction is defined as a Y1 direction, and the downward direction as a Y2 direction, and among the Z directions, the forward direction is defined as a Z1 direction, and the rearward direction as a Z2 direction. Unless otherwise noted, these directions represent directions of the manipulator 10 when it is of a neutral attitude. The definition of the above directions is for illustrative purpose only, and the manipulator 10 can be used in any orientations, e.g., it may be used upside down.

The working unit 16 comprises a distal-end working unit 12 for performing working operation, a connector 15 connected to an actuator block (actuator unit) 30 of the operation command unit 14, and an elongate hollow joint shaft 48 coupling the distal-end working unit 12 and the connector 15 to each other. When a predetermined action is performed on the actuator block 30, the working unit 16 can be separated from the operation command unit 14, so that the working unit 16 can be cleaned, sterilized, and serviced for maintenance.

The distal-end working unit 12 and the joint shaft 48, which are small in diameter, can be inserted into a body cavity 22 through a trocar 20 in the form of a hollow cylinder mounted in an abdominal region or the like of the patient. The distal-end working unit 12 is actuated by the operation command unit 14 to perform various surgical techniques to remove, grip, suture, or ligate (tie-knot) an affected part of the patient's body in the body cavity 22.

The operation command unit 14 includes a grip handle 26 gripped by hand, a bridge 28 extending from an upper portion of the grip handle 26, and the actuator block 30 connected to a distal end of the bridge 28.

As understood clearly from FIG. 1, a lower surface of the connector 15 abuts against an upper surface of the actuator block 30 with substantially no gaps therebetween, whereas the rear surface (surface facing the Z2 direction) of the connector 15 abuts against a front surface (surface facing the Z1 direction) of the bridge 28 with substantially no gaps therebetween. The lower surface of the connector 15 and the upper surface of the actuator block 30 lie in an XZ plane, and the rear surface of the connector 15 and the front surface of the bridge 28 lie in an XY plane. The left and right surfaces of the connector 15 and the left and right surfaces of the bridge 28 and the actuator block 30 make up a continuous YZ plane respectively, whereas the upper surface of the connector 15 and the upper surface of the bridge 28 respectively and continuously form a smooth curved surface. Owing thereto, the connector 15 is formed integrally and compactly with respect to the operation command unit 14, and moreover, since unnecessary irregularities in shape hardly exist at the region where the connector 15 and the operation command unit 14 are interconnected, operability is excellent.

As shown in FIG. 2, the grip handle 26 of the operation command unit 14 extends in the Y2 direction from the end of the bridge 28, and has a length suitable for being gripped by hand. The grip handle 26 has a trigger lever 32 as an input means, a composite input unit (finger-operated input unit) 34 and a switch 36. The grip handle 26 has a plurality of vent holes (pressure regulating mechanism) 41 defined in a lower end portion thereof. An LED (indicator) 29 is mounted on the upper surface of the bridge 28 at a location which can easily be viewed by the operator of the manipulator 10. A cable 62 has an end connected to the lower end of the grip handle 26 and an opposite end connected to a controller 45 (see FIG. 1). The grip handle 26 and the cable 62 may be connected to each other by a connector.

Structural and operational details of the operation command unit 14 will be described below. First, structural and operational details of the composite input unit 34 will be described below.

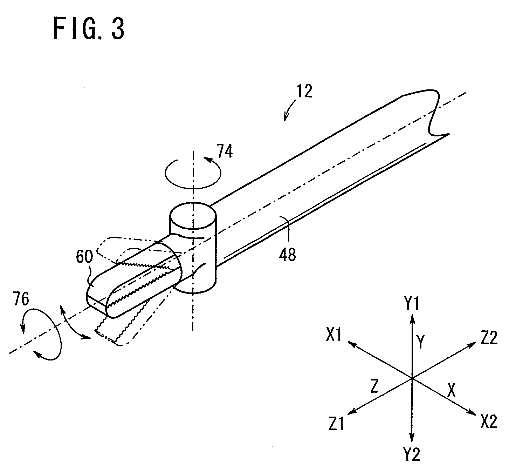

The composite input unit 34 serves as a composite input means for giving rotational commands in rolling directions (shaft rotating directions 76 in FIG. 3) and yawing directions (left and right directions 74 in FIG. 3) to the distal-end working unit 12. A trigger lever 32 serves as an input means for giving opening and closing commands to a gripper 60 (see FIG. 2) of the distal-end working unit 12. The switch 36 serves as an input means for selectively enabling and disabling the manipulator 10. The LED 29 serves as an indicator for indicating a controlled state of the manipulator 10. The LED 29 is of a size large enough to be easily visually recognizable by the operator, and yet is sufficiently small and light not to interfere with the operation of the manipulator 10. The LED 29 is located in a visually recognizable position substantially centrally on the upper surface of the bridge 28.

As shown in FIG. 3, the distal-end working unit 12 is mounted on the distal end of the joint shaft 48, and includes a plurality of pulleys with wires, not shown, wound therearound. The wires are also wound around respective pulleys 50a, 50b, 50c disposed in the connector 15 and disengageably connected to respective motors (actuators) 40, 42, 44 that are mounted in the actuator block 30. The distal-end working unit 12 is angularly movable about three axes when the pulleys are rotated about their own axes by the motors 40, 42, 44. These angular motions of the distal-end working unit 12 include a rotary motion in the directions 74 about a yaw axis (angularly movable mechanism, referred to as "yaw axis 74"), a rotary motion in the directions 76 about a roll axis (rolling mechanism, referred to as "roll axis 76"), and an opening and closing motion of the gripper 60 (angularly movable mechanism). The roll axis 76 rotates about an axis extending toward the distal end. When the yaw axis 74 is in a 0° position, the roll axis 76 rotates about the axis of the joint shaft 48.

In FIG. 3, the gripper 60 has a pair of openable/closable pinching members which are shown by the solid lines when closed and by the imaginary lines when opened. Though both of the pinching members of the gripper 60 are shown as being openable, only one of the pinching members may be openable.

The distal-end working unit 12 may be of the same mechanism as the working unit on the distal end of the medical manipulator disclosed in Japanese Laid-Open Patent Publication No. 2002-102248, for example.

The gripper 60 is angularly movable about the yaw axis 74 through an operating range of ±90° from a predetermined reference attitude. The gripper 60 is also angularly movable about the roll axis 76 through an operating range of ±180° from a predetermined reference attitude. The gripper 60 is openable through an angular range from 0 to 50°.

Since the yaw axis 74, the roll axis 76, and the gripper 60 can possibly cause a mutual interference, the controller 45 calculates an amount of interference and controls the wires to move back and forth to compensate for an interfering movement. In other words, the controller controls the wires such that when it moves one of the movable members, it prevents the other from moving into interference with the moved one.

Different tools including scissors, an electrosurgical knife, a microwave antenna, etc. are selectively available for use in place of the gripper 60 of the distal-end working unit 12, so that the gripper 60 can be replaced with a selected one of those tools. When the working unit 16 is removed from the actuator block 30, the motors 40, 42, 44 and the pulleys 50a, 50b, 50c (see FIG. 2) are returned to their respective reference positions so that the pulleys 50a, 50b, 50c will correctly engage the motors 40, 42, 44, respectively, when the working unit 16 will be mounted again on the actuator block 30. At this time, the yaw axis 74, the roll axis 76, and the gripper 60 are returned to their reference attitudes (0° position). When the yaw axis 74 is in the reference attitude, the gripper 60 is aligned coaxially with the joint shaft 48. When the roll axis 76 is in the reference attitude, the gripper 76 is in an intermediate angular position in the operating range of ±180°. When the gripper 76 is in the reference attitude, the pinching members thereof are closed.

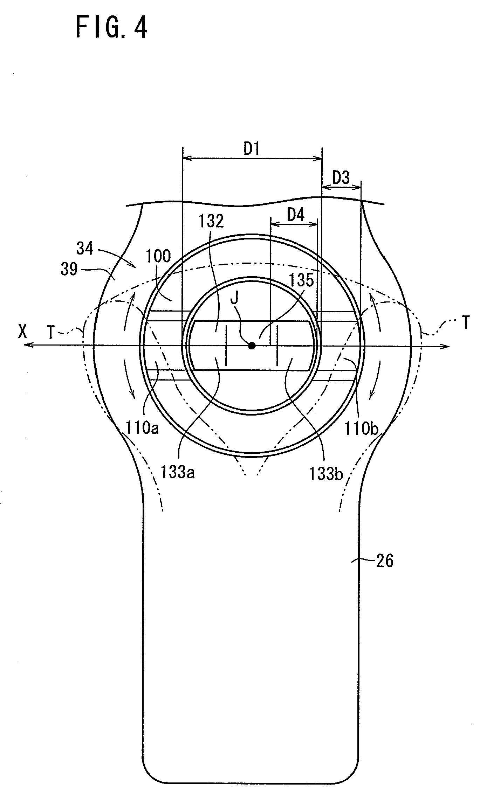

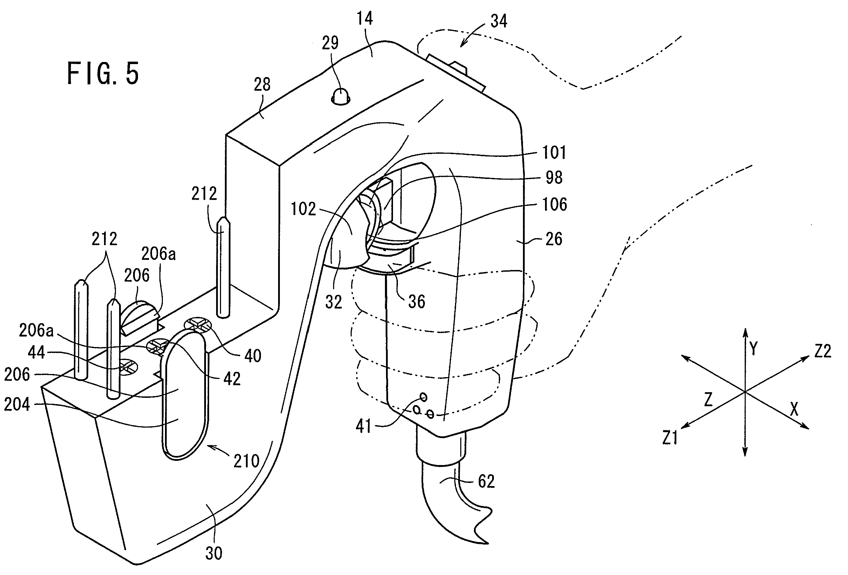

As shown in FIG. 4, the composite input unit 34 is of a circular shape when viewed in front elevation and is provided on a flat area 39 of the joint between the upper end of the grip handle 26 and the bridge 28. As can be seen from FIG. 5, the composite input unit 34 is disposed in a position where it can easily be operated by the thumb of the hand which is gripping the grip handle 26.

The flat area 39 is of a substantially annular shape that is larger in diameter than the composite input unit 34. When the composite input unit 34 is not to be operated, the operator, typically the surgeon, places the thumb on the flat area 39, so that the operator can firmly grip the grip handle 26 without touching the composite input unit 34. A normal line to the flat area 39 and the surface of the composite input unit 34 extends along a direction which lies substantially intermediate between the Z2 direction and the Y1 direction. Therefore, the operator can have the finger pad T of the thumb held naturally against the flat area 39 and the surface of the composite input unit 34.

The composite input unit 34 includes a shuttle ring 100 disposed in the flat area 39 and a pad 132 disposed in the shuttle ring 100. The shuttle ring 100 has an inside diameter D1 large enough to keep the pad 132 therein. The shuttle ring 100 is positioned within the movable range of the thumb of the hand that is gripping the grip handle 26 so that the shuttle ring 100 can easily be operated by the thumb. The shuttle ring 100 has a width D3 which is of such a dimension that the operator can apply the thumb appropriately onto the shuttle ring 100.

The shuttle ring 100 includes a pair of knobs 110a, 110b disposed on the shuttle ring 100 bilaterally (diametrically) symmetrically with an axis J thereof as a center. The knobs 110a, 110b slightly protrude away from the plane of the shuttle ring 100 for contacting with the finger pad T of the thumb.

The shuttle ring 100 is angularly movable about the axis J in an angular movable range of ±10° from a neutral position thereof. The angular movable range of the shuttle ring 100 should preferably be large enough to allow the shuttle ring 100 to move a certain distance for better operability, e.g., for entering delicate actions, and should preferably be kept within the movable range of the finger pad T of the thumb.

The pad 132 comprises a projection having upper and lower straight surfaces extending parallel to each other as viewed in front elevation and left and right arcuate ends which are convex radially outwardly. The left and right arcuate ends of the pad 132 have a radius of curvature such that the left and right arcuate ends are complementary in shape to the circular inner surface of the shuttle ring 100.

The pad 132 has a curved outer end surface facing outwardly which includes a central low flat facet 135 and a pair of left and right slanted facets 133a, 133b disposed one on each side of the central low flat facet 135. The left and right slanted facets 133a, 133b are gradually inclined outwardly away from the central low flat facet 135. Therefore, the central low flat facet 135 and the left and right slanted facets 133a, 133b can easily be distinguished by a tactile feel when the finger pad T of the thumb is placed on the pad 132. Each of the left and right slanted facets 133a, 133b has a width D4 which is of such a dimension that the operator can apply the thumb appropriately onto the shuttle ring 100.

When the shuttle ring 100 and the pad 132 are not operated by the thumb, they are automatically returned to their neutral position shown in FIG. 4 under the bias of resilient members, not shown.

Structural and operational details of the trigger lever 32 will be described below.

As shown in FIG. 5, the trigger lever 32 is disposed slightly below the bridge 28 and projects in the Z1 direction. The trigger lever 32 is disposed in a position where it can easily be operated by the index finger or middle finger of the hand that is gripping the grip handle 26.

The trigger lever 32 is operatively coupled to the grip handle 26 by an arm 98, and is movable toward and away from the grip handle 26. The arm 98 is operatively connected to a sensor, not shown, disposed in the grip handle 26. The distance that the trigger lever 32 has moved toward or away from the grip handle 26 is detected by the sensor, which supplies a signal representing the detected distance, to the controller 45.

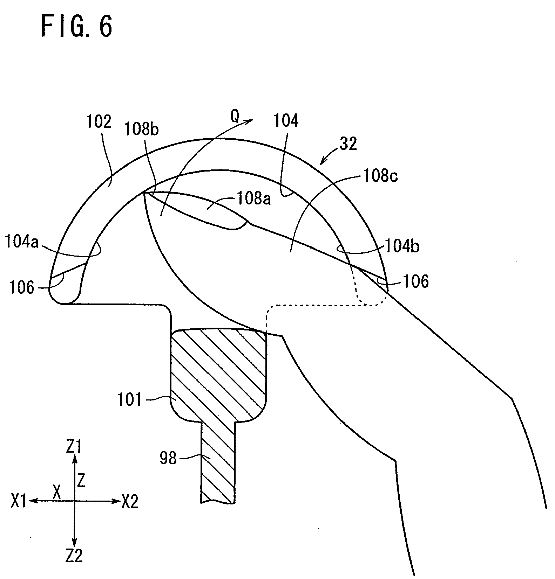

As shown in FIGS. 5 and 6, the trigger lever 32 comprises a pulling member 101 which can be pulled toward the grip handle 26 (i.e., in the Z2 direction) by the finger held against the pulling member 101, and a pushing member 102 which can be pushed away from the grip handle 26 in the Z1 direction by the finger held against the pushing member 102. The pushing member 102 is positioned in facing relation to the pulling member 101. The pushing member 102 has a cavity 104 defined in a surface thereof which faces the pulling member 101 in the Z1 direction. The pushing member 102 also has a pair of recesses 106 defined in the respective opposite ends thereof and communicating with the cavity 104 for allowing the finger to be smoothly inserted into the cavity 104.

The pulling member 101 is elongate in the Y directions and is of an arcuate shape as viewed in side elevation (see FIG. 2) so that the pulling member 101 can easily be engaged by the finger. The pulling member 101 is of such dimensions that the operator can hold the pad of the index finger or the pad of the middle finger extending distally from the second joint thereof (central joint), naturally against the pulling member 101. When the operator pulls the pulling member 101 toward the grip handle 26 (in the Z2 direction), the gripper 60 of the distal-end working unit 12 is closed in accordance with the distance that the pulling member 101 is pulled.

The pulling member 101 and the pushing member 102 are connected to each other at their upper and lower ends. Each of the pulling member 101 and the pushing member 102 is of a symmetrical shape with respect to a central axis thereof. The pulling member 101 and the pushing member 102 can be operated by the index finger or the middle finger of either the right hand or the left hand. Specifically, the index finger or the middle finger of either the right hand or the left hand can be inserted into the space between the pulling member 101 and the pushing member 102 from the right-hand or left-hand side of the trigger lever 32. The cavity 104 has a substantially hemispherical inner surface which can be slidingly contacted by the inserted fingertip to move the pushing member 102 accurately over small distances.

Specifically, as shown in FIG. 6, the operator may insert the portion of the index finger which extends distally from the first joint thereof (outermost joint) into the cavity 104, and hold a nail surface 108a, a nail tip 108b, or a finger back 108c of the index finger against the inner surface of the cavity 104. More specifically, the operator inserts the fingertip of the index finger into the cavity 104 while slightly bending the index finger at the first and second joints until the nail tip 108b contacts with the inner surface of the cavity 104. Then, the operator straightens the first and second joints to move the nail tip 108b in the direction indicated by the arrow Q in FIG. 6, pushing the inner surface of the cavity 104 in the Z1 direction.

When the inserted index finger is moved in the cavity 104 along the inner surface thereof from a farthest end 104a thereof to a nearest end 104b thereof, the trigger lever 32 is displaced in the Z directions by small distances depending on the movement of the finger, so as to perform delicate operations. When the pushing member 102 is pushed in the Z1 direction, the gripper 60 of the distal-end working unit 12 is opened depending on the distance that the pushing member 102 is pushed.

As shown in FIG. 6, the recesses 106 allow the user to insert the finger easily into the cavity 104 for better operability of the trigger lever 32. The recesses 106 and the openings of the cavity 104 are sized to provide a relatively small space just enough to allow the finger to be inserted into the cavity 104. When the finger is inserted into the cavity 104, the first joint of the finger is essentially held in contact with the pulling member 101. Therefore, after the operator has pushed the trigger lever 32, the operator can immediately pull the trigger lever 32 with almost no useless finger motion involved. As the operator can easily remove the finger out of the cavity 104, the operator can quickly go to another process after having stopped operating the trigger lever 32.

The trigger lever 32 is not normally resiliently biased to move toward or away from the grip handle 26. Though general trigger levers are normally resiliently biased to move away from the grip handle, since the trigger lever 32 is not resiliently biased to move toward or away from the grip handle 26, the operator can pull and push the trigger lever 32 with natural responses and does not find it fatiguing to operate the trigger lever 32 for a long period of time.

The trigger lever 32 is not in the form of a simple ring, but has the cavity 104 in the pushing member 102. Consequently, usability of the trigger lever 32 is not adversely affected by the size of the finger used therewith, and the trigger lever 32 can be operated equally by various different people as the operator, regardless of whether the operator is a male or a female.

Specifically, the operator with a thick index or middle finger may insert the finger into a central region of the cavity 104 in the Y directions because the central region of the hemispherical cavity 104 is relatively wide. Even though the thick finger is inserted in the cavity 104, it is not unduly pressed by the trigger lever 32 and the operator can operate the trigger lever 32 appropriately.

The operator with a thin index or middle finger may insert the finger into a region of the cavity 104 near its upper or lower end in the Y directions because the upper and lower ends of the hemispherical cavity 104 are relatively narrow. Even though the thin finger is inserted in the cavity 104, no undue gaps are made between the finger and the trigger lever 32 and the operator can operate the trigger lever 32 appropriately.

The operator may touch the trigger lever 32 with the finger in any ways not limited to the above-illustrated ways because the manner in which the operator uses the finger and the shape of the finger vary from operator to operator.

As described above, in the operation command unit 14 of the manipulator 10 according to the present embodiment, the pushing member 102 of the trigger lever 32 has the cavity 104 for receiving the fingertip. The pushing member 102 can be moved in small distances by the fingertip inserted in the cavity 104 for accurately opening and closing the gripper 60 or scissors of the distal-end working unit 12.

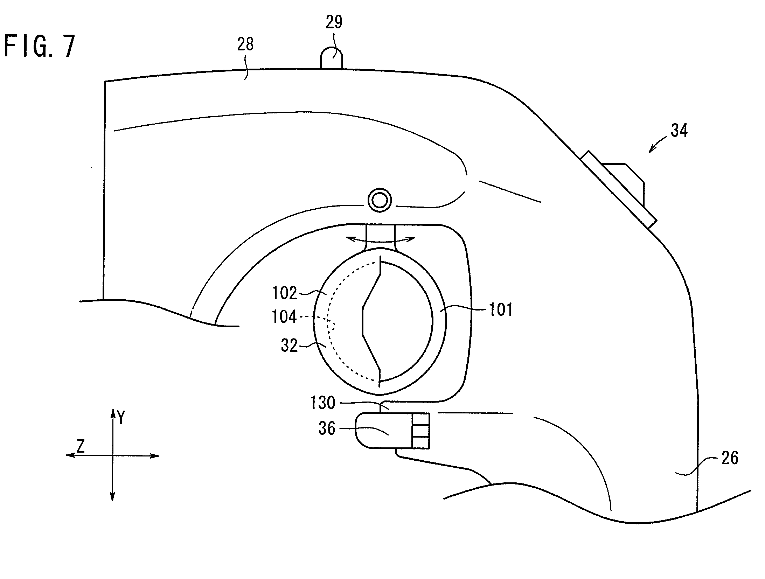

The arm 98 of the trigger lever 32 does not need to be connected to the grip handle 26. As shown in FIG. 7, the arm 98 of the trigger lever 32 may be pivotally connected to the bridge 28 for angular movement in a YZ plane. The pivoted trigger lever 32 is angularly movable toward and away from the grip handle 26. Alternatively, the arm 98 may be connected to the actuator block 30.

Structural and operational details of the switch 36 and the vent holes 41 will be described below.

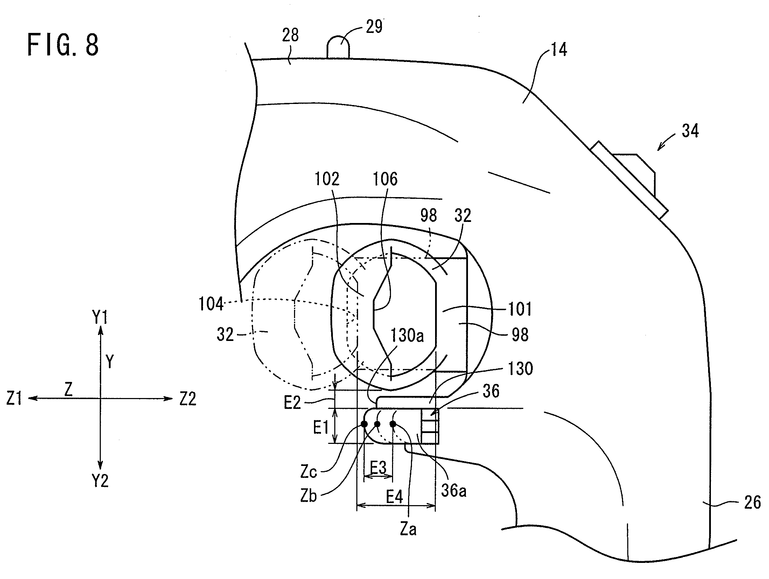

As shown in FIG. 8, the switch 36 serves as an operating mechanism movable toward and away from the grip handle 26. The trigger lever 32 and the switch 36 are disposed on the surface of the grip handle 26 on the Z1 direction side and are juxtaposed in the longitudinal directions (Y directions) of the grip handle 26. The switch 36 is disposed directly below the trigger lever 32 in the Y2 direction with a thin plate 130 interposed between the switch 36 and the trigger lever 32. The thin plate 130 extends from the grip handle 26 in the Z1 direction.

The switch 36 comprises an alternate switch having a trigger knob 36a. The switch 36 operates as follows: When the trigger knob 36a is pushed by the finger from a position Zc, which is indicative of an OFF position, to a changeover position Za which is spaced from the OFF position Zc in the Z2 direction, the switch 36 is locked in an ON state. When the trigger knob 36a is then released from the finger, it is held in a position Zb which is closer to the OFF position Zc than the changeover position Za. When the trigger knob 36a is pushed to the changeover position Za again, the switch 36 is released from the ON state into an OFF state. The trigger knob 36a is automatically returned to the OFF position Zc under the bias of a resilient member, not shown. When the switch 36 is repeatedly operated in this manner, it is automatically held in either the ON state or the OFF state, and the operator does not need to push the trigger knob 36a continuously to hold the switch 36 in either the ON state or the OFF state. The operator may operate the switch 36 only when it to switch between the ON state and the OFF state. When the operator does not operate the switch 36, the operator can operate the trigger lever 32. Accordingly, the switch 36 and the trigger lever 32 may be present closely together.

The trigger knob 36a projects to different positions when the switch 36 is in the ON state and the OFF state. Therefore, the operator can easily confirm the ON state and the OFF state of the switch 36 by seeing or feeling the trigger knob 36a.

The switch 36 serves to selectively enable and disable the manipulator 10, and to return the motors 40, 42, 44 to their origin when the manipulator 10 is disabled. Specifically, when the switch 36 is in the ON state, it enables the manipulator 10, and when the switch 36 is in the OFF state, it disables the manipulator 10. Enabled and disabled states of the manipulator 10 are indicated by the LED 29.

As shown in FIG. 8, the changeover position Za to switch between the ON state and the OFF state is preferably located more closely to the grip handle 26 in the Z2 direction than a distal end 130a of the plate 130. For shifting the switch 36 into either the ON state or the OFF state, the trigger knob 36a needs to reach the changeover position Za once. If the operator simply pulls the finger to push the trigger knob 36a, then the finger may be obstructed by the plate 130 and may fail to push the trigger knob 36a to the changeover position Za. Therefore, the operator is required to intentionally displace the finger slightly in the Y2 direction to avoid the plate 130. As a result, the switch 36 is prevented from being carelessly triggered into the ON state or the OFF state.

The trigger knob 36a is displaceable between the OFF position Zc and the changeover position Za by a distance E3 which is smaller than the distance E4 by which the pulling member 101 is displaceable in the Z directions. The operator can thus operate both the trigger lever 32 and the switch 36 with one finger without the need for shifting the hand that grips the grip handle 26, and hence find it easy to handle the manipulator 10.

The switch 36 is of a flat shape in the Y directions. As the switch 36 does not extend extensively along the grip handle 26 in the Y directions, it does not present an obstacle to the hand of the operator which grips the grip handle 26. The operator can easily operate both the trigger lever 32 and the switch 36 with the index finger. The switch 36 has a thickness E1 in the Y directions which may be in the range from 3 to 8 mm for better operability and strength. The trigger lever 32 and the switch 36 are spaced from each other in the Y directions by a distance E2 which may be in the range from 3 to 5 mm.

According to the present embodiment, as described above, the operation command unit 14 has the switch 36 and the trigger lever 32 which are present closely together, i.e., juxtaposed in the Y directions, so that they can easily be operated by the operator with one finger.

The operator may touch the switch 36 with the finger in any ways not limited to the above-illustrated ways because the manner in which the operator uses the finger and the shape of the finger vary from operator to operator.

The switch 36 may be a momentary switch. If the switch 36 is a momentary switch, then the switch 36 allows the manipulator 10 to perform a certain action while it is being pushed. Alternatively, the signal output from the momentary switch 36 may be processed according to a program such that the switch 36 is turned on when it is pushed an odd number of times, and turned off when it is pushed an even number of times.

As shown in FIGS. 2 and 5, the vent holes 41, e.g., three vent holes 41, are defined in the grip handle 26 near its lower end. Each of the vent holes 41 is of a diameter small enough to prevent water and dust from entering thereinto and large enough to allow air to flow therethrough to prevent the difference between the pressure inside the grip handle 26 and the pressure outside the grip handle 26 from becoming excessively large in a sterilizing process. For example, if there are two or more vent holes 41, each of the vent holes 41 may have a diameter ranging from 0.01 to 0.1 mm, and if there is only one vent hole 41, it may have a diameter ranging from 0.2 to 0.5 mm.

The grip handle 26 is of a hollow structure housing therein mechanisms combined with the composite input unit 34, the sensor for detecting an operating amount of the trigger lever 32, and the switch 36. These mechanisms and the sensor can be laid out in the grip handle 26 with large layout freedom, and can be protected from water, dust, and other foreign matter.

The operation command unit 14 including the grip handle 26 is sterilized each time a surgical operation involving the manipulator 10 is finished. According to the invention, the operation command unit 14 is sterilized by a plasma process. In the plasma process, the operation command unit 14 is placed in a chamber, and the chamber is evacuated. Thereafter, the chamber is filled with a hydrogen peroxide gas, and then is subjected to a high-frequency voltage applied thereto. When the chamber is evacuated, air contained in the hollow grip handle 26 flows out through the vent holes 41, thereby preventing an excessive differential pressure buildup between the inside of the grip handle 26 and the outside of the grip handle 26. Accordingly, expanding forces are not applied to the grip handle 26 under the internal pressure. Thus, the grip handle 26 is not required to be excessively high in mechanical strength, and hence may be light in weight and long in service life.

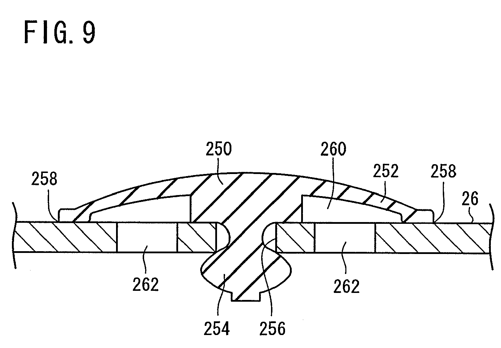

The grip handle 26 may be combined with another pressure regulating mechanism, rather than the vent holes 41, for preventing an internal pressure buildup in the hollow grip handle 26. For example, such another pressure regulating mechanism comprises a check valve (pressure-regulating valve) 250 shown in FIG. 9. The check valve 250 is made of a polymeric elastic material such as silicone rubber, acrylonitrile rubber, NBR, or the like. The check valve 250 is of a mushroom shape which is circular as viewed in plan, and has a skirt 252 and a central knob 254 integrally projecting from the center of the skirt 252. The central knob 254 is inserted and fixed in an attachment hole 256 defined in the grip handle 26. When the check valve 250 is mounted on the grip handle 26, the skirt 252 has its outer peripheral edge 258 elastically held against the surface of the grip handle 26, providing a space 260 between the skirt 252 and the grip handle 26. The grip handle 26 has a plurality of communication holes 262, each of a suitable diameter, defined therein around the central knob 254 in communication with the space 260.

Since the inside of the grip handle 26 and the space 260 are kept in communication with each other at all times through the communication holes 262, the same pressure is maintained in the inside of the grip handle 26 and the space 260. Usually, the inside of the grip handle 26 and the space 260 are hermetically sealed from the outside of the grip handle 26 by the check valve 250 against the entry of water and dust. When the difference between the pressure inside the grip handle 26 and the pressure outside the grip handle 26 becomes greater than a predetermined level in the sterilizing process, the skirt 252 is elastically expanded away from the grip handle 26 under the internal pressure in the grip handle 26, pushing the outer peripheral edge 258 off the surface of the grip handle 26, whereupon the internal pressure is released out of the grip handle 26 through the communication holes 262. As a result, an excessive differential pressure buildup is prevented from occurring between the inside of the grip handle 26 and the outside of the grip handle 26. The check valve 250 is small in size, light in weight, inexpensive to manufacture, and can be installed easily. The pressure regulating mechanism may alternatively be a spring-loaded check valve.

The connector 15 of the working unit 16 and the actuator block 30 of the operation command unit 14 are removably connected to each other by a joint mechanism. Structural and operational details of the joint mechanism will be described below.

As shown in FIGS. 1 and 2, the connector 15 is covered with a resin cover 37 and houses the driven pulleys 50a, 50b, 50c rotatably supported therein. The wires, not shown, are trained respectively around the pulleys 50a, 50b, 50c and extend through the hollow joint shaft 48 to the distal-end working unit 12.

The connector 15 also has two engaging teeth 200 disposed respectively on opposite side surfaces thereof and three fitting holes 202 defined therein which are open at a lower surface thereof. Of the three fitting holes 202, two are disposed near an end of the array of pulleys 50a, 50b, 50c in the Z1 direction and one near the opposite end of the array of pulleys 50a, 50b, 50c in the Z2 direction. The three fitting holes 202 extend in the Y1 direction.

The actuator block 30 houses therein the motors 40, 42, 44 corresponding to the three-degree-of-freedom mechanism of the distal end working unit 12, the motors 40, 42, 44 being arrayed in the longitudinal direction of the connector 15. The motors 40, 42, 44 have respective shaft ends positioned at the upper surface of the actuator block 30 for engagement with the respective pulleys 50a, 50b, 50c. The motors 40, 42, 44 are small in size and diameter, and the actuator block 30 which houses the motors 40, 42, 44 therein is of a flat compact shape. The actuator block 30 is disposed below an end of the operation command unit 14 in the Z1 direction. The motors 40, 42, 44 are energized under the control of the controller 45 based on actions made by the operator on the

### operation command unit 14 and the trigger lever 32.

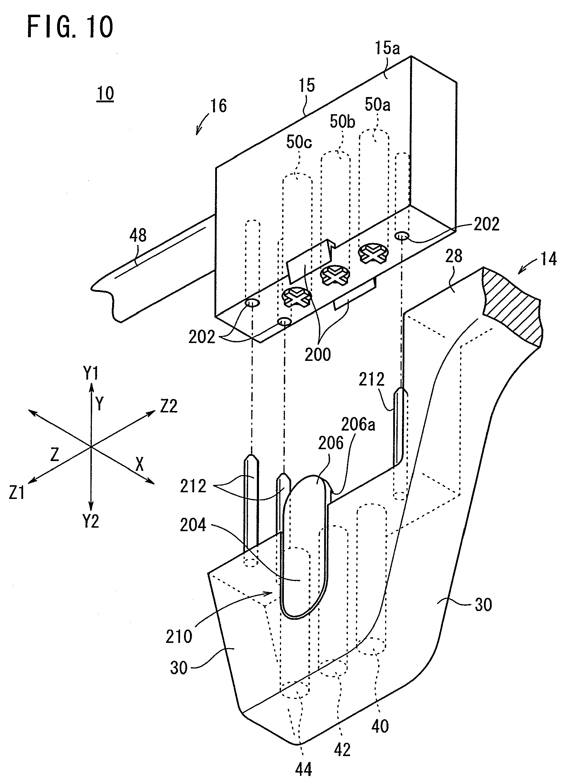

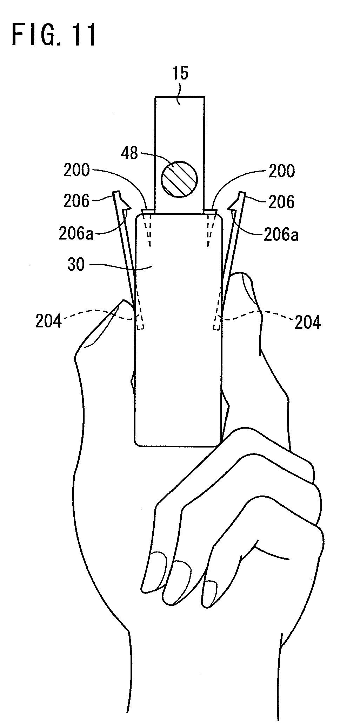

As shown in FIGS. 2 and 10, the actuator block 30 has two independent engaging fingers (engaging members) 210 for holding the connector 15, and three alignment pins 212 for positioning and holding the connector 15. In FIGS. 10 and 11, the cover 37 (see FIG. 1) is omitted from illustration for an easier understanding of the structure of the connector 15.

The engaging fingers 210 are disposed in symmetric positions on opposite side surfaces of the actuator blocks 30. The engaging fingers 210 include respective pusher surfaces (releasing members) 204 and respective levers 206 extending from the pusher surfaces 204 in the Y1 direction. The levers 206 project beyond the upper surface of the actuator block 30 in the Y1 direction and have tapered inner surfaces on their distal ends. The engaging fingers 210 are normally resiliently biased by resilient members, not shown, to displace the levers 206 inwardly toward each other.

The alignment pins 212 are mounted on the upper surface of the actuator block 30 in alignment with the respective fitting holes 202 in the connector 15. Specifically, two of the alignment pins 212 are disposed near an end of the array of motors 40, 42, 44 in the Z1 direction and one near the opposite end of the array of motors 40, 42, 44 in the Z2 direction. The alignment pins 212 extend in the Y1 direction. The two alignment pins 212 near the end of the array of motors 40, 42, 44 in the Z1 direction are spaced from each other in the X directions.

When the connector 15 is coupled to the actuator block 30, the alignment pins 212 are inserted respectively in the fitting holes 202 to position and hold the connector 15. Since the actuator block 30 has the three alignment pins 212, the connector 15 is supported by the actuator block 30 at the three points corresponding to the alignment pins 212 and is simply and reliably positioned with respect to the actuator block 30. As the three alignment pins 212 are not positioned in a linear array, the alignment pins 212 can hold the connector 15 stably against twisting forces applied in any directions.

The lower surface of the connector 15 and the upper surface of the actuator block 30 are held in face-to-face contact with each other (see FIG. 1), and the rear surface of the connector 15, i.e., a surface thereof which faces in the Z2 direction, and the front surface of the bridge 28, i.e., a surface thereof which faces in the Z1 direction, are held in face-to-face contact with each other (see FIG. 1). These surfaces are held in face-to-face contact with each other and the engaging fingers 210 are also effective to hold the connector 15 stably against twisting forces applied in any directions.

As shown in FIG. 2, the connector 15 has a connective structural member 15a with the fitting holes 202 defined therein. The cover 37 is mounted on the connective structural member 15a. The connective structural member 15a has a mechanical strength large enough for the connector 15 to be connected to the operation command unit 14. The alignment pins 212 have a height H1 greater than the height H2 of the connective structural member 15a, so that when the alignment pins 212 are inserted respectively in the fitting holes 202, the tip ends of the alignment pins 212 extend out of the fitting holes 202 and project slightly beyond the connective structural member 15a.

The height H1 of the alignment pins 212 may be at least one-half of the height H2 of the connective structural member 15a, preferably at least three-fifths of the height H2 of the connective structural member 15a, or more preferably greater than the height H2 of the connective structural member 15a. Therefore, the alignment pins 212 are sufficiently deeply fit in the respective fitting holes 202, thereby reliably holding the connector 15 on the actuator block 30.

As shown in FIG. 10, for connecting the connector 15 to the actuator block 30, the operator displaces the connector 15 in the Y2 direction toward the actuator block 30 in order to have the alignment pins 212 inserted respectively into the respective fitting holes 202. As the connector 15 is displaced further toward the actuator block 30, as shown in FIG. 11, the levers 206 of the engaging fingers 210 are displaced outwardly by the tapered inner surfaces which slide on the outer surfaces of the engaging teeth 200. When the lower surface of the connector 15 abuts the upper surface of the actuator block 30, the levers 206 snap back under the resiliency of the resilient members, bringing the wedges 206a into engagement with the engaging teeth 200. When the connector 15 is completely mounted on the actuator block 30, the levers 206 click to snap back and the connector 15 also produces sounds as it hits the actuator block 30, allowing the operator to confirm that the connector 15 is properly mounted on the actuator block 30.

When the connector 15 is connected to or removed from the actuator block 30, the rear surface (FIG. 1) of the connector 15 and the front surface of the bridge 28 are held in sliding contact with each other. As a consequence, the connector 15 can stably be connected to or removed from the actuator block 30.

As shown in FIG. 11, for removing the connector 15 from the actuator block 30, the operator presses the pusher surfaces 204 of the engaging fingers 210 simultaneously toward each other to tilt the levers 206 against the resiliency of the resilient members out of engagement with the engaging teeth 200.

The two independent engaging fingers 210 reliably hold the connector 15 in position on the actuator block 30 while the manipulator 10 is in operation. Even if one of the engaging fingers 210 is released from the corresponding engaging tooth 200, the other engaging finger 210 remains in engagement with the other engaging tooth 200, reliably holding the connector 15 connected to the actuator block 30.

The two independent engaging fingers 210 may be at least operationally independent of each other so that even when one of the engaging fingers 210 is pushed out of engagement with the corresponding engaging tooth 200, the other engaging finger 210 remains in engagement with the other engaging tooth 200. The engaging fingers 210 may be mechanically combined with each other in some ways insofar as they are operationally independent of each other. For example, the engaging fingers 210 may be biased by a common resilient member.

If the actuator block 30 has three or more independent engaging fingers 210, then the operator may possibly find it difficult to remove the connector 15 from the actuator block 30 alone. However, since the manipulator 10 has the two engaging fingers 210, the operator can easily remove the connector 15 from the actuator block 30 alone.

Inasmuch as the engaging fingers 210 are disposed in respective symmetric positions on the opposite side surfaces of the actuator blocks 30, the engaging fingers 210 are well balanced and are capable of reliably holding the connector 15. Since the actuator block 30 has the alignment pins 212 for fitting in the respective fitting holes 202 in the connector 15, the connector 15 is easily and reliably positioned with respect to the actuator block 30.

Since the connector 15 is reliably held by the three alignment pins 212, the engaging fingers 210 may act only as a means for holding the connector 15 against accidental removal, and hence may not be of excessively high mechanical strength.

In the operation command unit 14, the shafts of the motors 40, 42, 44 and the grip handle 26 extend substantially parallel to each other, and the grip handle 26 has its upper end connected to the actuator block 30 by the bridge 28 and its lower end connected to the cable 62.

Since the motors 40, 42, 44 and the grip handle 26 extend substantially parallel to each other, the operation command unit 14 does not have an unduly large width and hence is compact and easy to handle. As the cable 62 is connected to the lower end of the grip handle 26, the cable 62 is not liable to interfere with operations of the manipulator 10. The weight of the motors 40, 42, 44 and the forces from the cable 62 are distributed, and thus, the operator easily operates the manipulator 10 and is less subject to fatigue of the wrist of the hand that grips the grip handle 26. The forces from the cable 62 refer to the sum of its weight, tension, and frictional forces thereon, and are applied to the grip handle 26.

The trigger lever 32 is disposed between the actuator block 30 and the grip handle 26 and is movable toward and away from the grip handle 26. The composite input unit 34 is mounted on the upper end of the grip handle 26 remotely from the junction between the grip handle 26 and the actuator block 30. Accordingly, the trigger lever 32 can easily be operated by the index finger, for example, of the hand that grips the grip handle 26, and the composite input unit 34 can easily be operated by the thumb, for example, of the same hand. The trigger lever 32 and the composite input unit 34 are thus of high operability.

The motors 40, 42, 44, which are provided as a plurality of actuators, and the grip handle 26 lie in an array in the XY plane. Therefore, the operation command unit 14 is not unduly wide and hence is compact and easy to handle.

The upper surface of the bridge 28 which interconnects the upper end of the grip handle 26 and the actuator block 30 can easily be visually recognized by the surgeon while the surgeon is performing a surgical operation. Therefore, the upper surface of the bridge 28 is a suitable place for the LED 29.

Manipulators 10a, 10b, 10c according to first, second, and third modifications of the present invention will be described below. Those parts of the manipulators 10a, 10b, 10c which are identical to those of the manipulator 10 are denoted by identical reference characters, and will not be described in detail below.

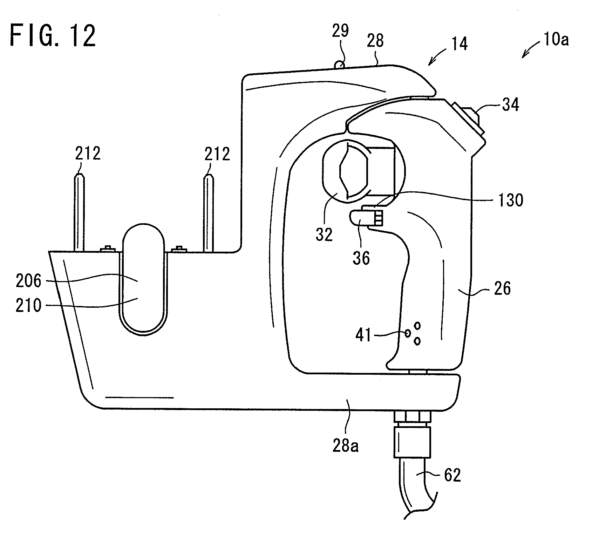

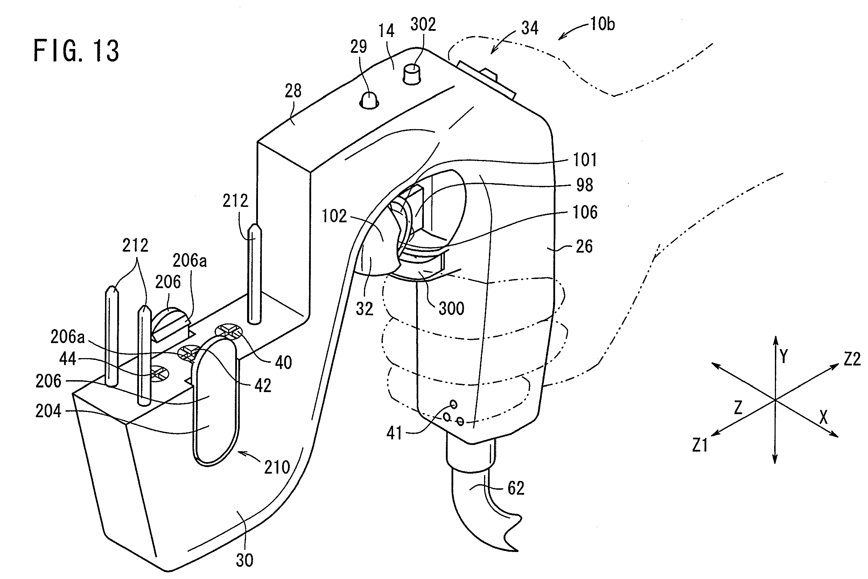

The grip handle 26 of the aforementioned manipulator 10 is integrally fixed to the bridge 28. As shown in FIG. 12, the manipulator 10a according to the first modification has a grip handle 26 angularly movably connected to a bridge 28 for angular movement in a predetermined angular range with respect to the bridge 28. The angularly movable grip handle 26 is capable of having a high degree of freedom for a gripping attitude thereof. The rotation angle between the grip handle 26 and the bridge 28 may be detected and used to generate an attitude command for the distal-end working unit 12. As shown in FIG. 12, the lower end of the grip handle 26 may be connected to the actuator block 30 by a lower grip 28a, so that the grip handle 26 is more reliably held. In FIG. 12 and also FIGS. 13 through 16, the working unit 16 is omitted from illustration.

As shown in FIG. 13, the manipulator 10b according to the second modification has a first switch 300 which is positioned at the same position as the switch 36 described above and a small second switch 302 disposed near the LED 29, the first switch 300 and the second switch 302 being provided in the operation command unit 14. The second switch 302 is slightly displaced away from the LED 29 in the Z2 direction.

The second switch 302 which is in the operation command unit 14 can easily be operated by the operator who grips the grip handle 26. However, the second switch 302 may not necessarily be provided in the operation command unit 14. Instead, the second switch 302 may be provided in the controller 45. Each of the first switch 300 and the second switch 302 comprises a momentary switch. The first switch 300 is of the same type as the switch 36 described above, i.e., a trigger lever type, and can easily be operated.

When the first switch 300 is pressed, the controller 45 recognizes an origin return mode for the roll axis 76, latches the origin return mode, and energizes the motors 40, 42, 44 to return only the roll axis 76 to the origin attitude (the middle attitude in the range of ±180°). The controller 45 energizes the motors 40, 42, 44 for the purpose of compensating for operational interferences between the axes. At this time, the yaw axis 74 and the gripper 60 remain in their attitudes.

The roll axis 76 allows the gripper 60 to make complex motions suitable for surgical techniques. As can be seen from FIG. 3, the angular displacement of the roll axis 76 may not easily be recognized depending on the attitude of the gripper 60. However, the manipulator 10b is convenient to use in this regard because only the roll axis 76 can be returned to its origin attitude by the switch 300 while the yaw axis 74 and the gripper 60 remain in their attitudes.

For example, if the roll axis 76 has already been turned 180° in a positive direction when the operator wants to turn the roll axis 76 in the positive direction, then the roll axis 76 cannot be turned additionally. In this case, the operator presses the first switch 300 to initiate the origin return mode to return the roll axis 76 to its origin attitude, i.e., to turn the roll axis 76 180° in a negative direction. Then, the roll axis 76 is angularly movable through the operating range of ±180°.

The operator can cancel the origin return mode at any time by pressing the first switch 300 again. When the operator presses the first switch 300 while in the origin return mode, the roll axis 76 is immediately stopped from angularly moving. At this time, the operator may press the first switch 300 for a small period of time, and hence can cancel the origin return mode quickly and reliably.

The first switch 300 may be operated in a modified way. For example, the controller 45 may initiate the origin return mode as long as the first switch 300 is pressed, and may stop the origin return mode when the first switch 300 is released.

The yaw axis 74 and the gripper 60 return to their origins when the operator presses the second switch 302. Specifically, when the operator presses the second switch 302, the controller 45 energizes the motors 40, 42, 44 to return the roll axis 76, the yaw axis 74, and the gripper 60 to their origin attitudes. Thereafter, the origin return mode is finished. The controller 45 initiates the origin return mode when the operator continuously presses the second switch 302 for 1 to 2 seconds.

The first switch 300 and the second switch 302 are not limited to momentary switches. One or both of the first switch 300 and the second switch 302 may be alternate switches.

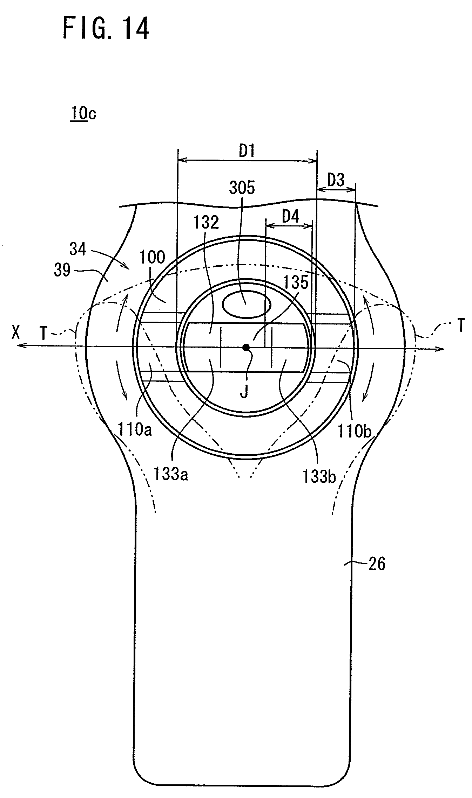

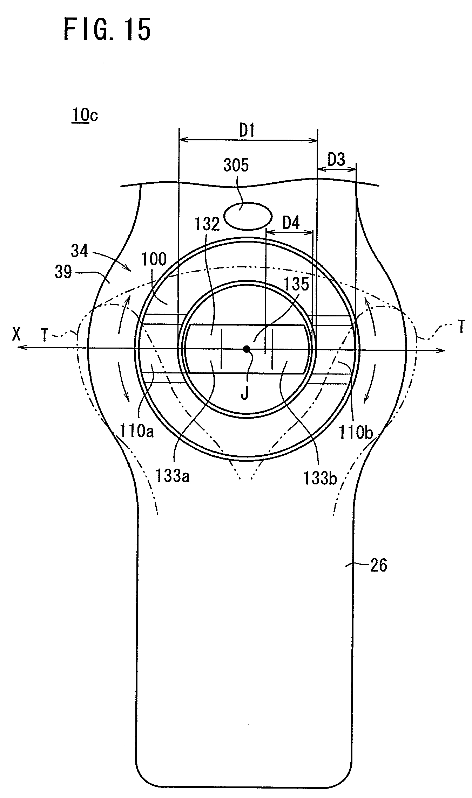

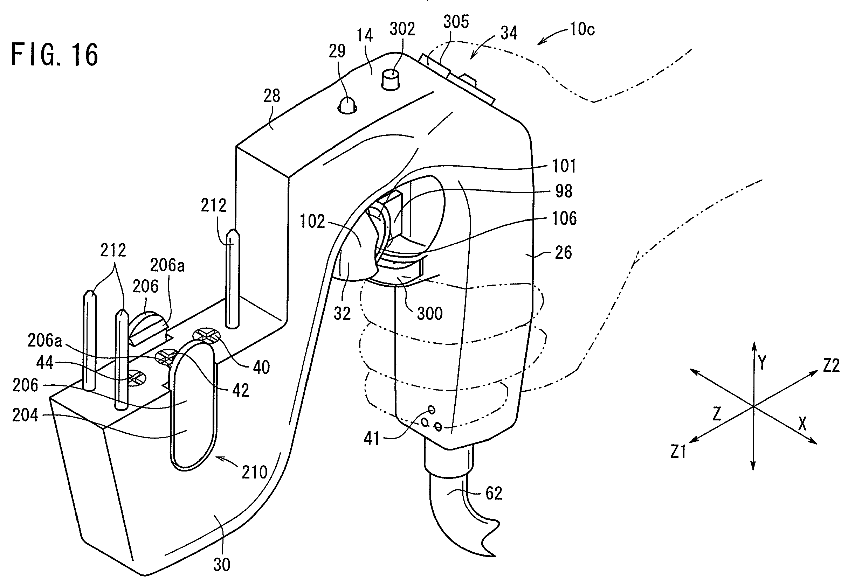

As shown in FIG. 14, the manipulator 10c according to the third modification has a third switch 305 added to the operation command unit 14 shown in FIG. 13. The third switch 305 is disposed within the shuttle ring 100 and positioned above the pad 132. The third switch 305 is disposed within the shuttle ring 100 for better operability. If the third switch 305 can be operated with ease during a surgical operation, it may be disposed outside the shuttle ring 100 and positioned closely above the shuttle ring 100, as shown in FIGS. 15 and 16. For example, the third switch 305 may be positioned between the composite input unit 34 and the second switch 302.

When the third switch 305 is pressed, the controller 45 recognizes an origin return mode for the yaw axis 74, latches the origin return mode, and energizes the motors 40, 42, 44 to return only the yaw axis 74 to the origin attitude (substantially parallel to the axis of the joint shaft 48).

The yaw axis 74 allows the gripper 60 to make complex motions suitable for surgical techniques. As can be seen from FIGS. 1 and 3, when the gripper 60 is tilted and not parallel to the axis of the joint shaft 48, the attitude of the gripper 60 needs to be changed for removing the distal-end working unit 12 from the patient because the distal-end working unit 12 would otherwise be caught in the trocar 20. In the manipulator 10c, only the yaw axis 74 can be returned to its origin attitude (i.e., an attitude coaxial with the joint shaft 48) simply by pressing the third switch 305 to return only the yaw axis 74 to its origin attitude, and thus, the operator can operate the manipulator 10c more easily.

The manipulators 10, 10a through 10c have been described as medical manipulators that are directly operated by the operator. However, the manipulators 10, 10a through 10c are also applicable to a remote control mechanism for performing medical operations on patients from a location remote therefrom through an electric communication means or the like.

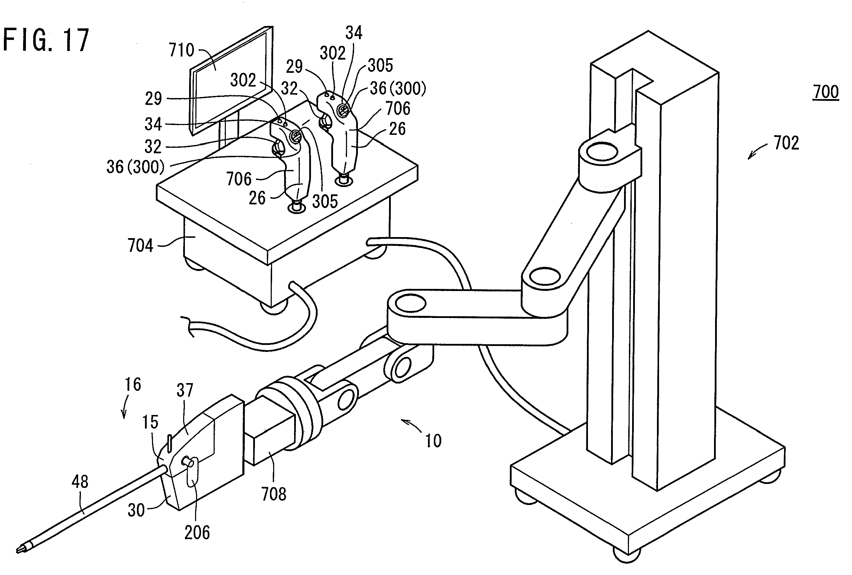

The working unit 16 has been described as connected to the operation command unit 14 that is manually operable. However, the working unit 16 may be applied to a surgical robot system 700 shown in FIG. 17, for example.

The surgical robot system 700 has an articulated robot arm 702 and a console (controller) 704. The working unit 16 is connected to the distal end of the robot arm 702. The same mechanism as that of the aforementioned actuator block 30 is provided at the distal end of the robot arm 702, thereby enabling connecting and actuating of the working unit 16. In this case, the manipulator 10 comprises the robot arm 702 and the working unit 16. The robot arm 702 may be a means for moving the working unit 16, and is not limited to an installed type, but, for example, may be of an autonomous movable type. The console 704 is away from the working unit 16 and the robot arm 702. The console 704 may be of a table type (control console), a control panel type, or the like.

The robot arm 702 should preferably have independent six or more joints (rotary shafts, slide shafts, etc.) for setting the position and orientation of the working unit 16 as desired. The actuator block 30 on the distal end of the robot arm 702 is integrally combined with a distal end portion 708 of the robot arm 702. The actuator block 30 has two independent levers 206 for locking the working unit 16.

The robot arm 702 is moved under operations of the console 704, and may be configured to move automatically according to a given program, or to move correspondingly to movements of joysticks (robot operating members) 706 mounted on the console 704, or to move by a combination of the program and the joysticks 706. The console 704 includes the function of the controller 45.

The console 704 includes the two joysticks 706 as an operation command unit exclusive of the actuator block 30 of the above operation command unit 14, and a monitor 710. Though not shown, the two joysticks 706 are capable of individually operating two robot arms 702. The two joysticks 706 are disposed in respective positions where they can easily be operated by the both hands of the operator. The monitor 710 displays information such as an image produced by an endoscope.

The joysticks 706 can be moved vertically and horizontally, twisted, and tilted, and the robot arm 702 can be moved depending on these movements of the joysticks 706. The joysticks 706 can be operated in the same manner as with the operation command units 14, by the trigger levers 32, the composite input units 34, and the switch 36 on the grip handles 26.

The joysticks 706 may have the first switches 300, the second switches 302, and the third switches 305 for individually resetting the distal-end working units 16.

The joysticks 706 may be master arms. The robot arm 702 and the console 704 may communicate with other via a communication means comprising a wired link, a wireless link, a network, or a combination thereof.

## Claims

1. A medical manipulator comprising:
a grip handle (26) for being gripped by a human hand;
a shaft (48) extending from the grip handle (26); and
a distal-end working unit (12) mounted on a distal end of the shaft (48);
the grip handle (26) being of a hollow structure and including at least one pressure-regulating mechanism
**characterized in that**
the pressure-regulating mechanism comprises a pressure-regulating valve (250) adapted to prevent a differential pressure buildup between the inside of the grip handle (26) and the outside of the grip handle (26).

2. The medical manipulator according to claim 1, wherein
the pressure regulating valve (250) is of a mushroom shape which is circular and has a skirt (252) and a central knob (254) integrally projecting from the center of the skirt (252), the central knob (254) being inserted and fixed in an attachment hole (256) defined in the grip handle (26) and the skirt (252) having its outer peripheral edge elastically held against a surface of the grip handle (26), providing a space (260) between the skirt (252) and the grip handle (26).

3. The medical manipulator according to claim 1, wherein
the pressure regulating valve is a spring-loaded check valve.

4. A method for sterilizing a grip handle (26) of a medical manipulator, the medical manipulator comprising:
the grip handle (26) for being gripped by a human hand;
a shaft (48) extending from the grip handle; and
a distal-end working unit (12) coupled to a distal end of the shaft,
wherein the grip handle (26) is of a hollow structure, has an attachment hole (256) defined on it, a plurality of communication holes (262) and has at least one pressure-regulating valve (250),
the method
being **characterized by** comprising the steps of:
placing the grip handle (26) in a chamber; and
sterilizing the grip handle (26) under reduced pressure in the chamber by a plasma process in which the chamber is evacuated and thereafter, the chamber is filled with a hydrogen peroxide gas, and then is subjected to a high-frequency voltage applied thereto,
said at least one pressure-regulating valve (250) allowing pressure regulation, and when the chamber is evacuated for sterilization, air contained in the grip handle (26) flows out trough the pressure-regulating valve (250).

## Patentansprüche

1. Medizinischer Manipulator mit:
einem Handgriff (26) zum Ergriffen werden durch eine menschliche Hand;
einem Schaft (48), der sich von dem Handgriff (26) erstreckt; und
einer Distalendarbeitseinheit (12), die an einem distalen Ende des Schafts (48) montiert ist;
wobei der Handgriff (26) von einer hohlen Struktur ist und zumindest einen Druckregelmechanismus hat,
**dadurch gekennzeichnet, dass**
der Druckregelmechanismus ein Druckregelventil (250) umfasst, das angepasst ist, einen Differenzialdruckaufbau zwischen dem Inneren des Handgriffs (26) und dem Äußeren des Handgriffs (26) zu verhindern.

2. Medizinischer Manipulator nach Anspruch 1, wobei
das Druckregelventil (250) von Form eines Pilzes ist, der kreisförmig ist und eine Schürze (252) und einen zentralen Knopf (254) aufweist, die einstückig von der Mitte der Schürze (252) vorspringen, wobei der zentrale Knopf (254) in eine Anbringungsbohrung (256) eingefügt und darin befestigt ist, die in dem Handgriff (26) definiert ist, und die äußere Randkante der Schürze (252) elastisch gegen eine Oberfläche des Hand-griffs (26) gehalten ist, wodurch ein Raum (260) zwischen der Schürze (252) und dem Handgriff (26) bereitgestellt ist.

3. Medizinischer Manipulator nach Anspruch 1, wobei
Das Druckregelventil ein federbelastetes Sperrventil ist.

4. Verfahren zum Sterilisieren eines Handgriffs (26) eines medizinischen Manipulators, wobei der medizinische Manipulator umfasst:
den Handgriff (26) zum Ergriffen werden durch eine menschliche Hand;
einen Schaft (48), der sich von dem Handgriff erstreckt; und
eine Distalendarbeitseinheit (12), die zu einem distalen Ende des Schafts gekoppelt ist,
wobei der Handgriff (26) von einer hohlen Struktur ist, eine darauf definierte Anbringungsbohrung (256) eine Mehrzahl Verbindungsbohrungen (262) und zumindest ein Druckregelventil (250) aufweist,
wobei das Verfahren durch das Umfassen der Schritte gekennzeichnet ist:
Platzieren des Handgriffs (26) in einer Kammer; und
Sterilisieren des Handgriffs (26) unter reduziertem Druck in der Kammer durch einen Plasmaprozess, in dem die Kammer evakuiert wird, und danach die Kammer mit einem Hydrogen-Peroxid-Gas gefüllt wird, und dann einer darauf angelegten Hochfrequenzspannung ausgesetzt ist,
wobei das zumindest eine Druckregelventil (250) eine Druckregelung gestattet, und wenn die Kammer zum Sterilisieren evakuiert ist, in dem Handgriff (26) enthaltene Luft durch das Druckregelventil (250) ausströmt.

## Revendications

1. Manipulateur médical comprenant :
une poignée de saisie (26) pour être saisie par la main de l'homme ;
un arbre (48) s'étendant à partir de la poignée de saisie (26) ; et
une unité de travail d'extrémité distale (12) montée sur une extrémité distale de l'arbre (48) ;
la poignée de saisie (26) ayant une structure creuse et comportant au moins un mécanisme de régulation de pression
**caractérisé en ce que**
le mécanisme de régulation de pression comprend une soupape de régulation de pression (250) adaptée pour empêcher une remontée de pression différentielle entre l'espace intérieur de la poignée de saisie (26) et l'espace extérieur de la poignée de saisie (26).

2. Manipulateur médical selon la revendication 1, dans lequel
la soupape de régulation de pression (250) est en forme de champignon qui est circulaire et a une jupe (252) et un bouton central (254) faisant saillie d'un seul tenant à partir du centre de la jupe (252), le bouton central (254) étant inséré et fixé dans un trou de fixation (256) défini dans la poignée de saisie (26) et la jupe (252) ayant son bord périphérique externe maintenu élastiquement contre une surface de la poignée de saisie (26), fournissant un espace (260) entre la jupe (252) et la poignée de saisie (26).

3. Manipulateur médical selon la revendication 1, dans lequel
la soupape de régulation de pression est un clapet de non-retour à ressort.

4. Procédé pour stériliser une poignée de saisie (26) d'un manipulateur médical, le manipulateur médical comprenant :
la poignée de saisie (26) pour être saisie par la main de l'homme ;
un arbre (48) s'étendant à partir de la poignée de saisie ; et
une unité de travail d'extrémité distale (12) couplée à une extrémité distale de l'arbre,
dans lequel la poignée de saisie (26) a une structure creuse, a un trou de fixation (256) défini sur celle-ci, une pluralité de trous de communication (262) et a au moins une soupape de régulation de pression (250),
le procédé étant **caractérisé en ce qu'**il comprend les étapes qui consistent :
à placer la poignée de saisie (26) dans une chambre ; et
à stériliser la poignée de saisie (26) sous pression réduite dans la chambre par un traitement au plasma où la chambre est vidée et par la suite, la chambre est remplie d'un gaz de peroxyde d'hydrogène, et est ensuite soumise à une tension haute fréquence appliquée à celle-ci,
ladite au moins une soupape de régulation de pression (250) permettant la régulation de pression, et lorsque la chambre est vidée pour la stérilisation, l'air contenu dans la poignée de saisie (26) s'écoule à travers la soupape de régulation de pression (250).
